# EUROPEAN PATENT SPECIFICATION

(11) **EP 0 808 782 B1**
(45) Date of publication and mention of the grant of the patent: **16.04.2003**
(21) Application number: 95942213.0
(22) Date of filing: 28.12.1995
(51) Int. Cl.: B65F 1/16, A61B 19/02

(54) **WASTE CONTAINER WITH CLOSURE AND OPENING TOOL THEREFOR**
RESTSTOFFBEHÄLTER MIT VERSCHLUSS SOWIE ÖFFNER
CONTENEUR DE RESIDUS AVEC FERMETURE, ET OUTIL D'OUVERTURE

(43) Date of publication of application: 26.11.1997
(73) Proprietor: Sanypick, S.A., 28814 Daganzo (ES)
(72) Inventor: GONZALEZ MEJIAS, David, 28220 Majadahonda (ES)
(74) Representative: Carpintero Lopez, Francisco
(86) International application number: ES9500156
(87) International publication number: WO97024280

(56) References cited:
- EP-A- 0 168 877
- ES-B- 2 024 033
- ES-T- 2 066 479
- ES-Y- 1 004 600
- US-A- 3 297 193
- US-A- 4 349 120

## Description

### OBJECT OF THE INVENTION

The present invention relates to a waste - containing case with closure and hinge system and particularly the means with which the lid of a case is provided in order for it to be articulately mounted on the mouth of said container, and for said lid to be sealed, the above based upon a perimetric edge of the lid having a pair of wings that are mounted upon a bolt of the container which constitutes the hinge or articulation system of the lid proper, which is further perimetrically provided with a skirt in which special wings are formed to be locked in appropriate openings provided to such end in a perimetric wing of the case proper.

The invention also relates to an implement allowing the lid and the body to be disengaged once the case is closed, i.e. the case to be opened, in the event of its contents having to be inspected.

### BACKGROUND OF THE INVENTION

Some waste-containing cases require a sealing system and further that once the appropriate lid is closed, the same be locked in order to prevent not only that it may be opened but to moreover any manipulation whatsoever tending to open said lid.

Containers of this kind are clearly designed for special waste, namely waste originating in hospitals or elsewhere which may be contaminating or even result in accidents or injuries, for instance syringes, sanitary materials, chemical products used in medicine, and in general waste of any other kind whatsoever requiring the container to be sealed, because of its bad smell or the risk of contamination or accident, ruling out any chance of the case housing said products being opened.

European patent number 0168877 describes a container with a closure system based upon its lid being provided with a perimetric skirt with outwardly arcuately and divergently projecting flanges that are inserted by resilient deformation in windows purposely provided in an inner perimetric wing located at the top mouth of the actual container, such that once the lid flanges extend beyond said container windows, they are retained due to their resilient springback, whereupon there is no chance of opening taking place.

The closure system described in said European patent 0168877 does not however include lid hinge means enabling the lid to be opened and not definitively closed until the container is full of waste products.

Furthermore, the flanges constituting the means for locking the lid after it is definitively closed can be manipulated and the lid opened by manually effected resilient deformation.

In conclusion, although the lid of European patent 0168877 meets the requirement of effecting a seal, and the closure achieved may indeed be deemed to be effective against potential attempts to open it, its drawback is nevertheless that there are no hinge means enabling the lid to be opened and closed to place the waste, before it is definitively closed, nor indeed are there suitable means to fully preventing manipulation and attempted opening of the lid after it is definitively closed as aforesaid.

European patent EP-A-0 326 799 describes a receptacle to contain disposable products wherein the receptacle on which a covering lid is fixed and this container has a mechanism to receive and hold this lid with which it establishes practicably hermetic closure.

### DESCRIPTION OF THE INVENTION

The system subject hereof, which applies to all containers having a lid for certain waste products as explained in the preceding section, is particular in that the lid is provided with a system hinging it to the case, enabling the lid to be opened as many times as desired before it is definitively locked, for the hinge system allows the lid to be opened and closed, even if closure is not definitive, since the lid will fit on the mouth of the case without being closed and definitively locked unless the actual user so desires.

In particular, the closure system of the invention is based upon the lid having its corresponding perimetric skirt provided with a pair of wings arranged parallel to one another and having a slotted window or deep groove in which a bolt crosswise thereto and provided in the actual case or container is positioned, said bolt to be arranged between two brackets projecting perpendicularly from the lateral surface of the case, close to its mouth.

A means is thus obtained for mounting and hinging the lid, which is moreover provided with a number of flanges formed on the perimetric skirt, said flanges having a sloped projection that allows them to be easily inserted in windows purposely provided in a perimetric wing of the case, and being locked when such projections extend beyond said windows, although when they do not so extend beyond the same, the lid may be opened and closed without being locked.

As for the perimetric wing emerging from the lateral surface of the case, said wing containing the windows for locking the flanges on the perimetric skirt of the lid, such has a crosswise upward and downward expansion which defines an upright sector perpendicular to the horizontal sector in which the said windows are provided, the above in order for the upright sector to constitute a means preventing the insertion of tools with which the closure means could potentially be prised to open the lid.

It should also be noted in this respect that right above the perimetric wing of the case, the wall of such case is substantially weakened on its inner surface and thus it is the case that is deformed, specifically its mouth is radial contracted, in a lid engagement operation and until such engagement is definitively achieved. This deformation of the mouth of the case is intermittent, at each of the flanges on the lid, since in addition to an inner weakening of the mouth of the case, strengthening ribs are located on its outside around the windows on the perimetric wing of the case body.

The invention also deals with an implement that, as aforesaid, allows the case to be opened once it has been definitively closed in order that its contents may be inspected. Said implement comprises a ring the shape and size of which are suited to the periphery of the body, close to the perimetric wing of said body, which it approaches from the bottom of the case body, said ring having a plurality of teeth, their number and position matching those of the windows on the perimetric wing, each tooth defining an inner inclined plane to internally deform the flanges on the lid, and an inner ledge for it to be axially displaced with respect to the body.

### DESCRIPTION OF THE DRAWINGS

In order to provide a fuller description and contribute to the complete understanding of the characteristics of this invention, a set of drawings is attached to the specification which, while purely illustrative and not fully comprehensive, shows the following:

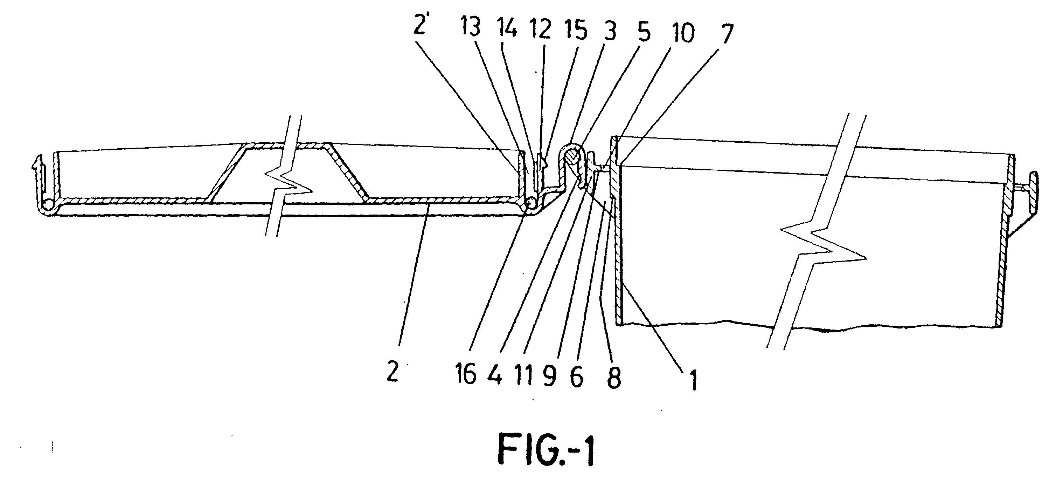

Figure 1.- Is a side elevation sectional view of the top, namely the mouth, of a case on which the relevant closure lid is mounted, in an open position, showing the actual hinge means.
Figure 2.- Is a close-view of the lid hinge system, with the lid in a non-definitive closed position, i.e. without the flanges provided thereon locked in the relevant windows of the body.
Figure 3.- Is a close-view resembling that of the preceding figure, albeit with the lid in the definitive closed position, i.e. with its flanges locked in the windows provided on the perimetric wing of the case.
Figure 4.- Is a side view of the lid, showing the locking flanges and the two wings constituting the lid hinge means to the relevant case or container in which said lid is applicable.
Figure 5.- Is a partial perspective close-view of the mouth of the body, showing the strengthening ribs around the weakened areas.
Figure 6.- Is a sectional sequence of the various case closing stages, showing the resilient deformation and subsequent springback of the case body.
Figure 7.- Is a partial side elevation close-view of the case opening implement.
Figure 8.- Is a sectional close-view of said implement.
Figure 9.- Is the implement of the preceding figure at the case body deformation stage, for the lid to be disengaged.

### PREFERRED EMBODIMENT OF THE INVENTION

With reference to the figures aforesaid, the case comprising the closure and hinge system of the invention consist of a case (1) of the type containing certain waste products that must be closed with the relevant lid (2) when full, the definitive closure having to be seal which cannot be opened by undue manipulation.

Now then, the hinge system comprises a pair of lateral wings (3) on the actual lid (2), said wings having a slotted window or deep groove (4) in which a bolt (5) is positioned, located crosswise between both wings (3) and which is provided on the actual case (1), which bolt (5) will be located, for instance, between a pair of brackets (6) provided on the actual lateral surface of the case (1). The bolt (5) will logically be arranged closed to the mouth of the case (1), such mouth having a pair of ledges (7) and (8), the first one being an inner ledge and the second one an outer ledge, their function to be explained hereinafter.

Furthermore, the lateral surface close to the actual top edge of the mouth of the case (1) has a perimetric wing (9) provided with windows (10), such wing extending into another wing (11) crosswise thereto, which will result in an outer upright stiffening portion (11) and an inner horizontal portion (9) where the windows (10) are precisely provided.

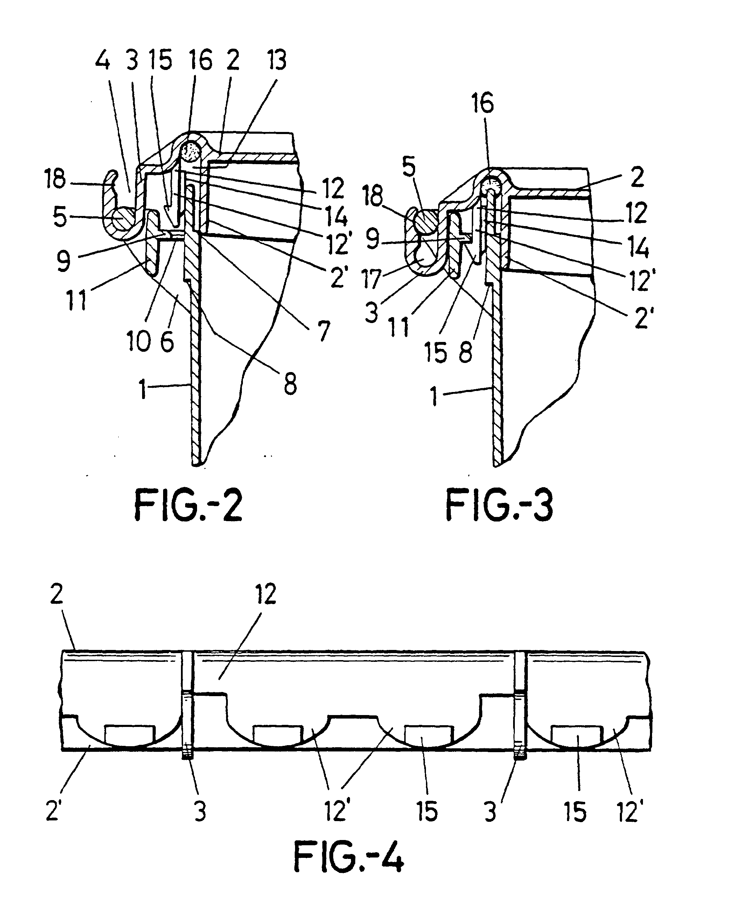

For its part, the lid (2) has a perimetric skirt (12) defining with another inner skirt (2') a groove (13) which with the lid (2) closed will constitute the means for positioning the top sector of the mouth of the actual case (1), as shown in figures 2 and 3. The skirt (12) has arcuate flanges (12') having a claw-like projection or heel (15) and the flanges (12') are internally provided with strengthening ribs (14) on their underside. The heel (15) defines a slope and a ledge to enable insertion and locking of the perimetric wing (9) of the case (1) in the windows (10). A sealing gasket (16) is provided on the bottom of the groove (13).

The arcuate configuration of the edge of the flanges (12') at the ends of the perimetric skirt (12) of the lid brings about a self-centering effect when the lid (2) is being closed over the mouth of the case (1).

As for the window (4) for positioning the bolt (5) constituting the hinge pin of the lid (2) proper, such has two distinct areas, one between the bottom (17) of such groove, and another one being an area (18) close to the opening of the groove (4) proper, thereby for the lid to remain stable, upon being mounted on the bolt or hinge pin (5) in any of such areas (17) and (18), due to their configuration.

In accordance with the characteristics described above, the open lid (2) will be located with the bolt (5) on the deepest area (17) of the groove (4) of the wings (3) of the lid (2) proper.

This position allows the lid (2) to be mounted and swung with respect to the case (1), and opened and closed without the closed position, as shown in figure 2, being definitive, i.e. the top sector of the mouth of the case (1) will be partly positioned in the groove (13) but not fully, and the projections or heels (15) of the flanges (12') will not therefore enter the windows (10) and there will therefore be no definitive locking, thereby allowing waste to be placed in the container (1) until it is full.

Definitive closure takes place when the container (1) is full, or when desired, to which end from the provisionally closed position shown in figure 2, the lid (2) is pushed down, thereby for the bolt (5) to move to the relevant position at the area (18) of the window (4) of the wings (3) forming part of the hinge system, in which position the flanges (12') will logically have been downwardly displaced upon the lid (2) being lowered and the projections or heels (5) will cross through the windows (10) and be locked, upon extending beyond the same, on the ledge defined externally on each of the projections or heels (15), whereupon the lid (2) will be definitively closed and may not be opened, for not even the flanges (12') may be manipulated, since this will be prevented by the transverse perimetric wing (11) emerging perpendicularly to the wing (9) where the windows (10) housing the locking heels (15) are provided.

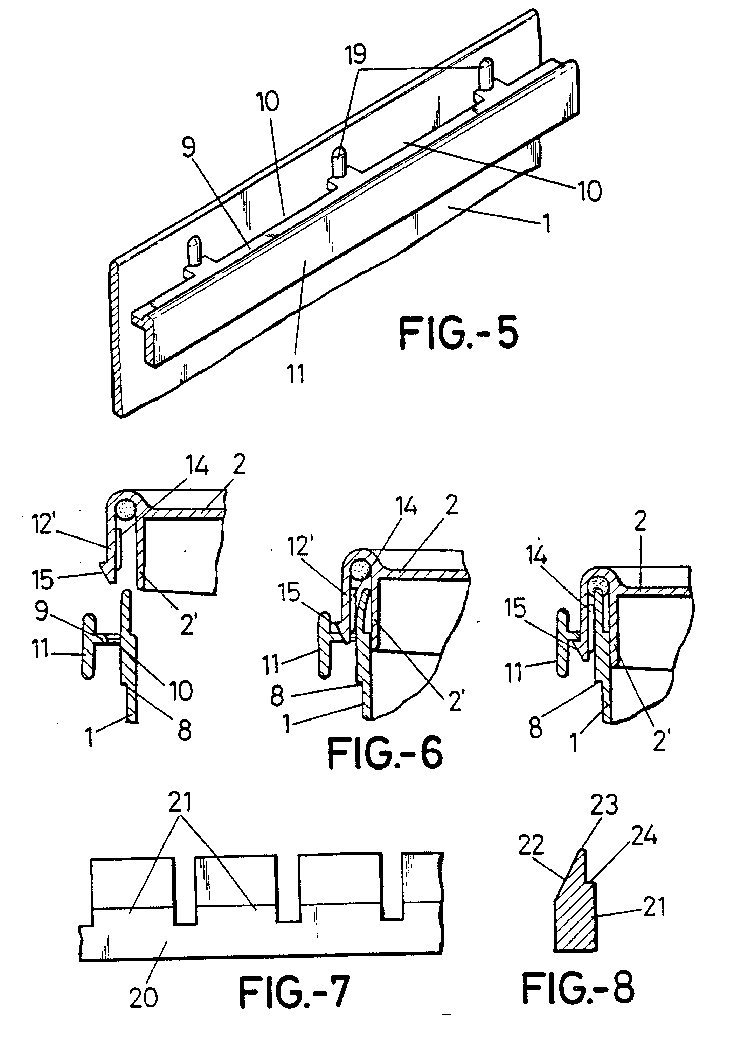

This operation takes place due to a resilient deformation of the mouth of the body (1), as shown particularly in the operative sequence of figure 6, to which end the mouth of the body (1) is weakened above the inner ledge (7) aforesaid, thereby for a lesser thickness to allow said resilient deformation, which takes place zonally, specifically at each of the flanges (12') on the lid, since the mouth of the body (1) in this weakened sector is stiffened by means of ribs (19), as may be clearly seen in figure 5, around the windows (10).

In this definitively closed position, the top edge of the mouth of the case (1) will push against the seal (16) provided on the bottom of the groove (13), as shown in figure 3, thereby establishing a total seal preventing the exit of smells.

The double ledge (7) and (8) on the lateral surface, both inside and outside the container (1), allows the lid to be self-centered in the groove (13), the perimetric inner skirt (2') on the actual lid assisting this, as shown in figures 2 and 3.

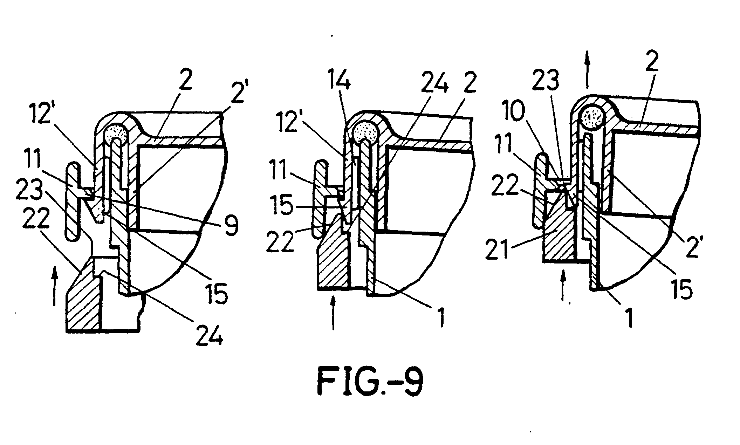

Although the case is designed so that, once it is definitively sealed, since full, it cannot be opened, it has been provided that if its contents should eventually need to be inspected, it may be opened, specifically with the assistance of the implement shown in figures 7 and 8, which in turn acts as shown in figure 9.

More specifically, said implement comprises a ring (20) capable of receiving therewithin the body (1) of the case, said ring (20) moving axially from the bottom of the case towards its mouth, the ring having a plurality of teeth (21), their number and position matching that of the windows (10) where the flanges (12') are locked, each of said teeth (21) being provided with an outer inclined plane (22) designed to impinge upon lower part of the transverse upright wing (11) of the case body (1) to internally deform the flanges (12') and thereby allow the heels (15) to be released from the wing (9), for its front (23) impinges on the inclined plane of said heels (15), also pushing them inwards, and finally an inner ledge (24) pushes the front of said flanges (12') to cause the lid to be definitively disengaged from the body (1).

We feel that the description need not be extended any longer for any expert in the art to have grasped the full scope of the invention and the advantages it offers.

The materials, shape, size and layout of the elements may be altered provided that this entails no modification of the essential features of the invention.

The terms used to describe the invention herein should be taken to have a broad rather than a restrictive meaning.

## Claims

1. A waste-containing case with closure and hinge system , being applicable in cases (1) of the kind having a closure lid (2) that must be locked to the case in the definitive position of closure as such to render opening thereof impossible, adjusting the top edge of the case (1) proper in a perimetric groove purposely formed on the actual lid (2), the groove having a sealing gasket (16) that is pressed by said top edge of the mouth of the case (1) for the same to be sealed, **characterised in that** the side surface of the lid (2) is provided with wings (3) having a slotted window (4), in which a bolt (5) provided on the case (1) and lying close to the outer lateral surface of said case (1) is arranged crosswise acting as a hinge pins, said lid (2) having a number of arcuate flanges (12') projecting from the lower edge of a perimetric skirt (12), which flanges (12') are provided with a claw-like heel (15) constituting the locking means when the lid (2) is definitively closed on the case (1), whereupon said projections or heels (15) cross through windows (10) provided on a perimetric wing (9) of the lateral surface of the case (1).

2. A waste-containing case with closure and hinge system ,as in claim 1, **characterised in that** the window (4) for mounting and hinging the lid (2) to the case (1) has two areas (17) and (18) at different heights, in either of which the bolt (5) may be stably positioned, and thus when the latter is positioned in the deepest area (17) the lid may be opened and closed as many times as desired to place waste in the case (1), whereas when the bolt (5) is positioned in the outermost area (18), the lid (2) will take up the definitively closed position, the heels (15) crossing through and being locked in the holes (10) of the perimetric skirt (9) of the case (1).

3. A waste-containing case with closure and hinge system , as in preceding claims, **characterised in that** the perimetric wing (9) of the case (1) where the windows (10) are provided for the heels (15) on the flanges (12') of the lid (2) to pass and be locked, has a transverse upright wing (11) located externally to define a means preventing access of lever tools to attempt to open the case after the lid (2) proper is definitively closed, the wall of the case (1) being substantially weakened on its inner face and following a ledge (7) right above the perimetric wing (9) in order that the lid be engaged by a resilient contraction of the mouth of the body.

4. An implement for inspection opening a waste-containing case fitted with the closure and hinge system of claim 3, **characterised by** comprising a ring (20) of suitable shape and size to adapt to the outside of the body (1) of the case, reaching such body from its bottom end, which ring (20) has a plurality of teeth (21), their number and position matching those of the windows (10) of the perimetric wing (9) of the body (1) of the case, each of such teeth (21) being provided to have a slope (22) or outer inclined plane through which to impinge on the lower part of the transverse upright wing (11) as the implement is moved up, having a front (23) impinging upon the inclined plane defined on the heels (15) on the flanges (12') of the lid (2) and an inner ledge (21) for pushing such flanges and ejecting the lid after they are deformed.

## Patentansprüche

1. Reststoffe enthaltender Behälter mit einem Verschluß- und Scharniersystem, das bei jenen Behälterarten(1) anwendbar ist, die mit einem Verschlußdeckel (2) ergänzt werden, der in der endgültigen Position des Verschlusses selbst bezüglich des Behälters verriegelt bleiben muss, um sein Öffnen unmöglich zu machen, wobei sich der obere Rand des Behälters (1) selbst in eine umkreisende Rille, die in dem Deckel (2) selbst dafür ausgebildet ist, anpasst, in die Rille, in der eine hermetisch dichtende Dichtung (16) liegt, auf die dieser obere Rand der Öffnung des Behälters (1) drückt, um den hermetisch dichten Schluss zu erwirken, **dadurch gekennzeichnet, dass** an der Seitenfläche des Deckels (2) Flügel(3) vorgesehen sind, die mit einem mit einer Nut versehenen Fenster (4) ausgestattet sind, in dem ein Bolzen (5), der zum Behälter (1) gehört und nahe der äusseren Seitenfläche des erwähnten Behälters (1) vorgesehen ist, querförmig positioniert ist, der wie ein Scharnier wirkt, wobei besagter Deckel (2) eine Reihe von bogenförmigen Nasen (12') aufweist, die vom inneren Rand einer Umkreisungsschürze (12) herausragen, Nasen (12') die mit einem Absatz (15) der Art eines Zapfens ausgestattet sind, der das Verriegelungselement beim endgültigen Verschluß des Deckels (2) auf dem Behälter (1) bildet, wenn die erwähnten Vorsprünge oder Absätze (15) durch Fenster (10) geführt werden, die an einem umkreisenden Flügel (9), der zur Seitenfläche des Behälters (1) gehört, vorgesehen sind.

2. Reststoffe enthaltender Behälter mit einem Verschluß- und Scharniersystem nach dem Patentanspruch 1, **dadurch gekennzeichnet, dass** das Fenster (4) zur Montage und zum gelenkigen Anschluss des Deckels (2) in Bezug auf den Behälter (1), über zwei Bereiche (17) und (18), in unterschiedlicher Höhe, verfügt, wo der Bolzen (5) in jeder der beiden Positionen in stabiler Form positioniert werden kann, so dass dessen Positionierung in der tiefsten (17) Stellung erlaubt, dass der Deckel so oft gewünscht wird, geöffnet und geschlossen werden kann, um Reststoffe in den Behälter (1) zu deponieren, während in der Position des Bolzens (5) in dem weiter aussenliegenden Bereich (18), der Deckel (2) die endgültige Verschlußposition einnimmt und die Absätze (15), in Bezug auf die Öffnungen (10) des umkreisenden Flügels (9) des Behälters (1), durchgeführt und verriegelt sind.

3. Reststoffe enthaltender Behälter mit einem Verschluß- und Scharniersystem nach vorhergehenden Patentansprüchen, **dadurch gekennzeichnet, dass** der umkreisende Flügel (9) des Behälters (1), wo die Fenster (10) für das Einführen und Verriegeln der zu den Nasen (12') des Deckels (2) gehörenden Absätze (15) ausgebildet sind, über einen transversalen und vertikalen Flügel (11) verfügen, der aussen angeordnet ist, um ein Element zu bilden, das nach dem endgültigen Verschliessen des Deckels (2) den Zugang mit Hilfe von Hebelwerkzeugen verhindert, um ein Öffnen des Behälters herbeizuführen, wobei die Wand des Behälters (1) auf der Innenseite und nach einer Abstufung (7), unmittelbar über dem umkreisenden Flügel (9), merklich abgeschwächt ist, so dass beim Aufsetzen des Deckels eine elastische Kontraktion der Öffnung des Körpers erfolgt.

4. Werkzeug zum Öffnen, für die Inspektion eines mit dem Verschluß-und Scharniersystem nach dem Patentanspruch 3 ausgestatteten Reststoffbehälters, **dadurch gekennzeichnet, dass** es aus einen Ring (20) besteht, der in seiner Form und seine Abmessungen geeignet ist, um sich aussen an den Körper (1) des Behälters anzupassen und zu diesem von seinem unteren Ende aus gelangt, ein Ring (20) der eine Vielzahl von Zähnen (21) aufweist, die in ihrer Anzahl und Stellung mit den Fenstern (10) des umkreisenden Flügels (9) des Körpers (1) des Behälters übereinstimmen, wobei vorgesehen ist, dass jeder Zahn (21) über eine äussere Rampe (22) oder geneigte Ebene verfügt, über die er auf den unteren Bereich des transversalen und vertikalen Flügels beim Hochgehen des Werkzeugs einwirkt, mit einer Einfallstirn (23) auf den auf den Absätzen (15) der Nasen (12') des Deckels(2) definierten schrägen Ebene und mit einer inneren Abstufung (21) zum Zwecke des Stossens auf besagte Nasen, um den Deckel nach deren Formveränderung abzuheben.

## Revendications

1. Conditionnement contenant des résidus avec un système de fermeture et de charnière applicable à ce type de conditionnements (1) qui sont complétés par un couvercle de fermeture (2) qui, en position définitive de la fermeture elle-même doit être enclenché sur le conditionnement pour empêcher son ouverture, où le bord supérieur du propre conditionnement (1) s'adapte sur une cannelure périmétrale composée à cet effet sur le propre couvercle (2), cannelure où existe un joint d'herméticité (16) sur lequel ce bord supérieur de l'embouchure du conditionnement (1) exerce une pression pour obtenir sa fermeture hermétique. Ce conditionnement se **caractérise par le fait qu'**à la surface latérale du couvercle (2) l'on a prévu des ailes (3) équipées d'une fenêtre fendue (4) sur laquelle est positionné transversalement un boulon (5) faisant partie du conditionnement (1) et prévu en situation proche de la surface latérale externe dudit conditionnement (1) qui agit comme une charnière. Ce couvercle (2) possède une série d'oreilles arquées (12') dépassant du bord inférieur d'un pan périmétral (12), oreilles (12') munies d'un talon (15) en guise de doigt qui constitue le moyen d'enclenchement lors de la fermeture définitive du couvercle (2) sur le conditionnement (1), quand lesdits rebords ou talons (15) traversent des fenêtres (10) prévues sur une aile périmétrale (9) correspondant à la surface latérale du conditionnement (1).

2. Conditionnement contenant des résidus avec système de fermeture et de charnière, d'après la revendication 1, **caractérisé par le fait que** la fenêtre (4) de montage et de charnière du couvercle (2) sur le conditionnement (1) possède deux zones (17) et (18) à différente hauteur, sur n'importe laquelle des deux on peut positionner de façon stable le boulon, de sorte sa position dans la zone la plus profonde (17) permette l'ouverture et la fermeture du couvercle autant de fois qu'on le voudra pour déposer des résidus dans le conditionnement (1), tandis qu'à la position du boulon (5) à la zone la plus externe (18), le couvercle (2) occupera la position de fermeture définitive, les talons (15) traversant les orifices (10) de l'aile périmétrale (99 du conditionnement (1) et étant enclenchés dedans.

3. Conditionnement contenant des résidus avec système de fermeture et de charnière d'après les revendications précédentes, **caractérisé par le fait que** l'aile périmétrale (9) du conditionnement (1) où se trouvent les fenêtres (10) de passage et d'enclenchement des talons (15) correspondant aux oreilles (12') du couvercle (2), possède une aile transversale et verticale (11), qui est située à l'extérieur pour former un moyen rendant impossible l'accès d'outils à levier pour tenter de procéder à l'ouverture du conditionnement après la fermeture définitive du propre couvercle (2), la paroi du conditionnement (1) étant sensiblement affaiblie au détriment de sa face interne et après un échelonnement (7), immédiatement au-dessus de l'aile périmétrale (9), de sorte que lors de l'accouplement du couvercle il se produit une contraction élastique de l'embouchure du corps.

4. Outil d'ouverture pour inspection d'un conditionnement conteneur de résidus muni du système de fermeture et de charnière de la revendication précédente, **caractérisé par le fait qu'**il est constitué par une bague (20), dont la forme et les dimensions sont adéquates pour s'adapter à l'extérieur du corps (1) du conditionnement, avec accès à ce dernier à partir de son extrémité inférieure, bague (20) munie d'un certain nombre de dents (21), dont le nombre et la position coïncident avec les fenêtres (10) de l'aile périmétrale (9) du corps (1) du conditionnement. Il a été prévu que chacune de ces dents (21) possède une rampe (22) ou plan incliné extérieur, grâce à la quelle elle tombe sur la partie inférieure de l'aile transversale et verticale lors de la montée de l'outil, avec un front (23) d'incidence sur le plan incliné défini aux talons (15) des oreilles (12') du couvercle (2) et avec un échelonnement interne (21) de poussée sur lesdites oreilles pour expulsion du couvercle après leur déformation
